# EUROPEAN PATENT SPECIFICATION

(11) **EP 3 076 904 B1**
(45) Date of publication and mention of the grant of the patent: **30.10.2019**
(21) Application number: 14830596.4
(22) Date of filing: 04.12.2014
(51) Int. Cl.: A61F 2/46, A61F 2/34, A61F 2/36, A61F 2/30

(54) **DEVICE TO EVALUATE CHARACTERISTICS OF A PATIENT FOR THE PURPOSE OF CORRECT APPLICATION OF A PROSTHESIS TO THE SAME**
VORRICHTUNG ZUR BEWERTUNG DER MERKMALE EINES PATIENTEN ZUR KORREKTEN ANWENDUNG EINER PROTHESE
DISPOSITIF PERMETTANT D'ÉVALUER LES CARACTÉRISTIQUES D'UN PATIENT CONCERNANT LA MISE EN PLACE CORRECTE D'UNE PROTHÈSE SUR LEDIT PATIENT

(30) Priority: 05.12.2013 IT VR20130271
(43) Date of publication of application: 12.10.2016
(73) Proprietor: Aeron S.r.l., 35134 Padova (IT)
(72) Inventor: GARRO, Carlo, 35010 Campodoro (Padova) (IT); GARRO, Damiano, 35010 Campodoro (Padova) (IT)
(74) Representative: Feltrinelli, Secondo Andrea
(86) International application number: PCT/IB2014/066593
(87) International publication number: WO 2015/083116

(56) References cited:
- EP-A2- 0 807 426
- EP-A2- 0 807 426
- WO-A1-03/094803
- AT-U1- 12 124
- US-A- 5 645 607
- US-A- 5 645 607
- US-A1- 2004 267 372
- US-A1- 2004 267 372
- US-B1- 7 425 214
- US-B1- 7 425 214

## Description

### TECHNICAL FIELD OF THE INVENTION

The present invention relates to a device for the detection and measurement of physical/structural characteristics of a patient to whom a hip prosthesis must be prepared, more particularly, a trial neck device and a device for measuring the femoral anteversion angle, the angle of anteversion of the acetabulum and/or of the cotyle and/or acetabular and or cotyloid inclination angle.

### DESCRIPTION OF THE PRIOR ART

Hip femoral prosthetic systems can present different configurations, but they are all arranged in such a way as to position the centre of joint rotation in a position as anatomical as possible for the respective patient.

In implementing a hip prosthesis, among the various purposes that one should set oneself and that a surgeon must achieve, there is, of course, to restore the limb length, joint stability, as well as the movement range and muscle balance, and this by restoring the horizontal offset or, in other words, reconstructing as faithfully as possible the original anatomical parameters, which may have been altered, for example, due to the onset and development of arthrosis, arthritis or due to trauma suffered by the patient or for other conditions. As it is known, by offset it is intended the distance between the centre of rotation of the joint head and the extension line of the diaphyseal or anatomical axis of the femur.

The femoral prosthetic components may have different configurations, thus each having a respective neck-shaft angle of the so-called "neck" component, standard or so-called "high offset" stems, necks of different lengths, joint heads with different lengths and diameters, and so on. Even the cotyle or the so-called "insert" can be of different types and configurations from one device to another, for example with or without positionable anti-dislocation, inclined, eccentric, protruded, retentive, "shoulders" or "roofs" etc., so that adaptation can be obtained for different situations and morphologies.

During surgery, the surgeon, whether (s)he has made or not a preoperative plan, will test the various options available to ensure that the basic parameters are guaranteed for the patient, including stability, leg length, range of displacement or "range of motion" and muscular balance.

These tests are usually performed through several repeated reduction and dislocation manoeuvres of the joint, by inserting a "femoral trial rasp" or trial rasp into the femur and then changing on the femoral trial rasp the various available trial necks and combining with these the various lengths of trial joint heads; every configuration change and check inevitably involves a joint dislocation and reduction manoeuvre, resulting in wasted time, increased surgical exposure times and increased surgical damage to the joint itself and to the muscles involved. On the basis of the outcome of the performed tests, the surgeon will then choose the final implant configuration.

This procedure is applied in combination with every intervention or surgical access way, as well as for any type of prosthesis, for both traditional techniques and techniques involving the use of "navigators" or robots and for minimally invasive techniques; in the latter case, in particular, the purpose is to minimise the surgical joint damage, in order to obtain a quicker postoperative functional recovery that is easier for the patient.

US7425214B1, US5645607A, US2004267372A1 and WO03094803A1 teach solutions according to prior art.

### OBJECTS OF THE INVENTION

The main object of the present invention is to provide a new device for the detection and, optionally, the measurement, in the course of the implementation of a hip prosthesis, of the physical/structural characteristics of a patient to whom a hip prosthesis must be made.

Another object of the present invention is to provide a new trial neck device.

In connection with an aspect of the invention a device is provided according to claim 1.

In connection with another aspect of the invention a kit is provided according to claim 15.

Dependent claims refer to preferred and advantageous embodiments of the invention.

### BRIEF DESCRIPTION OF THE DRAWINGS

Other characteristics and advantages of the invention will be more apparent from the description of embodiments of devices, illustrated only by way of example in the accompanying drawings in which:
- Figure 1 is a side view with sectional parts of a device according to the present invention;
- Figure 2 is a view similar to Fig. 1 of another embodiment of a device according to the present invention;
- Figure 3 is a view similar to Fig. 1 of a further embodiment of a device according to the present invention;
- Figure 4 is a comparative schematic view of different operating positions of the device of Fig. 3;
- Figures 5 and 6 are front views in respective operating positions of another example of a device according to the present invention;
- Figures 7, 8 are perspective views slightly from above and from one side of the device of Figures 5 and 6;
- Figure 9 is a perspective view slightly from above and from another side of the device of Figures 5 and 6;
- Figure 10 is a side view of the device of Figures 5 and 6;
- Figure 11 is a perspective view slightly from above of a device for a kit according to the present invention;
- Figure 12 is a view from above of the device of Fig. 11;
- Figure 13 is a view similar to Fig. 12 with parts shown in transparency;
- Figure 14 is a side view with parts in transparency of the device of Fig. 11;
- Figure 15 is an exploded view of the device of Fig. 11;
- Figures 16 and 17 are front and side views, respectively, of the devices of the group of Figs. 1, 2, 3 and 11 during one assembly phase;
- Figure 18 is a front view with parts shown in transparency of devices according to the present invention during one assembly phase;
- Figures 19 and 21 are front and side schematic views, respectively, of a femur in which a device is inserted according to the present invention;
- Figures 20 and 22 are front and top schematic views, respectively, of a pelvis;
- Figures 23 and 24 are exploded views of an embodiment of trial neck device according to the present invention similar to the device of Figure 3; and
- Figures 25 to 32 are side views in respective operating positions of the device of Fig. 23.

In the accompanying drawings the same parts or components are indicated by the same reference numerals.

### EMBODIMENTS OF THE INVENTION

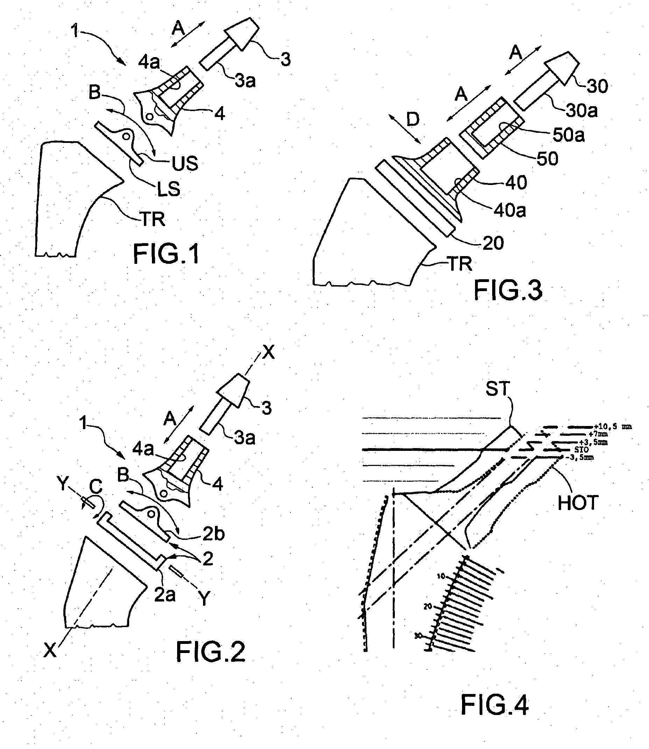

With reference to Figures 1 to 4, there is shown a trial neck device 1 for intraoperative measurement of the physical/structural characteristics of a patient for the realization of a hip prosthesis including a base component 2 intended to be constrained to a trial rasp TR or to a final modular femoral prosthetic component (i.e. the one either inserted or to be inserted and fixed in the femur of the patient) and a terminal or apical component 3 constrained to the base component 2 and intended to be housed proximally to or in the vicinity of the cotyle of a patient, for example, within the so-called head or small-head component to be inserted into the cotyle or in the the so-called "insert" component.

Adjustment means are then provided for adjusting the distance (see arrow A in the Figure) and/or the angular position (see arrow B in the Figure) of the apical component 3 with respect to the base component 2. The apical component 3 or rather the side surface of same can be, for example, configured as a cone or truncated cone, with a tapered cross section in the moving-away from the base component 2 direction.

Furthermore, a device according to the present invention is suitable for detecting and/or measuring the characteristics with joint reduced, that is to say with the femur and/or cotyle in their anatomical positions. Therefore, the adjustment means can also be operated with a reduced joint. In this regard, the device can comprise an engagement seat or recess for the insertion and operation of the adjustment means through a tool, such as a screwdriver or an Allen key or another means of transmission of motion. Such engagement seat is open in use towards the front and/or rear of the device and, if desired, extends about an axis in the antero-posterior direction, so that it is engageable through a surgical tool starting from the front side of the patient, both for the case of a right hip and for the case of a left hip, and thus equally as easily with joint reduced, so as to avoid repeated joint dislocation, adjustment of the device and joint reduction manoeuvres.

More particularly, the apical component 3 is slidingly mounted with respect to the base component 2 so as to be moveable apart and closer with respect to it. In this regard, the apical component 3 can have a stem portion 3a intended to slidingly engage in a seat 4a of an intermediate group, comprising a first intermediate component 4, which intermediate group 4, on the one side, is constrained to the base component 2 and, on the other side, to the apical component 3. The stem portion 3a can, according to a variant, be slidingly mounted within a seat formed in the base component 2. The apical component 3 can be locked at different operating positions with respect to the base component 2 or intermediate component 4.

Alternatively, the first intermediate component 4 may have a stem or shank for sliding engagement with a respective seat of the apical component 3. Furthermore, the first intermediate component 4a could be slidingly engaged with the base component 2.

According to the example illustrated in Figure 1, the intermediate group 4 has at least one portion pivoted to the base component 2, preferably about an axis intended to be substantially parallel to an antero-posterior direction (that is to say, from the front to the rear of the patient treated) so as to assume different neck-shaft angles. Alternatively, the intermediate group 4 could be pivoted to the apical component 3.

Basically, the device 1 has a base body 2, for example substantially flat, provided with a lower surface, in use, LS engageable with the trial rasp TR or with a final prosthetic component, and an upper surface, in use, US, and is hinged, preferably at the upper surface US, to the intermediate component 4. The intermediate component 4 defines instead the seat 4a with main extension, in use, towards the cotyle in which the stem portion 3a of the apical component 3 is slidingly engaged. Naturally, the hinge axis or axes of the intermediate component 4 to the base component 2 is/are substantially orthogonal to the main extension axis of the seat 4a or rather of the stem 3a of the apical component 3.

A device such as that illustrated in Figure 1 can provide, for example, different neck-shaft angles in addition to the ability to change the distance of the apical component 3 from the base component 2.

With reference now to Figure 2, a device similar to that illustrated in Figure 1 is shown, but in which the base component 2 has a first portion 2a intended to be adapted and fixed to a trial rasp TR or to a final prosthetic component, as well as a second portion 2b, preferably hinged or pivoted in the lateral-medial direction (see arrow C) to the first portion 2a. More particularly, the second portion 2b is pivoted to the first portion 2a about an axis y-y orthogonal to both the direction of displacement x-x of the apical component 3, and the axis of rotation (axis entering the sheet) of the intermediate group with respect to the base component 2, or better still, with respect to the second portion 2b.

The second portion 2b, together with the first intermediate element 4 and the apical component 3 constrained to it, can thus assume different inclinations in the antero-posterior direction, in use, which will enable to arrange the elements 3 and 4 with an anteverted, in axis or retroverted orientation. In this regard, in modular neck prostheses, to correct any positioning errors of the femoral stem, if the stem is excessively anteverted, retroverted modular necks are provided and, if the stem is excessively retroverted, anteverted necks are provided.

The device according to Figure 2 can therefore be disposed in different trims, with different neck-shaft angles (see arrow B) and different neck lengths (see arrow A) and also different orientations of the neck device in the antero-posterior, i.e. anteverted, in axis and retroverted directions (see arrow C). To modulate the offset and/or length of the neck device, the length of the apical component 3 can be changed or the length of the prosthetic neck can be changed while maintaining a fixed length of the apical component.

Referring now to Figure 3, a trial neck device 1 is illustrated for the measurement of the physical/structural characteristics of a patient for the realization of a prosthesis comprising a base component 20 adaptable and fixable to a trial rasp TR or to a final prosthetic component, as well as an apical component 30 and an intermediate group comprising one or more, preferably two intermediate components, a first intermediate component 40 constrained to the base component 20 and a second intermediate component 50 constrained, on the one side, to the first intermediate component 40 and, on the other side, to the apical component 30.

More particularly, the base component 20 may have a slide portion, while the first intermediate component 40 has a sliding block portion intended to slidingly engage in the slide portion of the base component 20, so as to allow a shift in the medial-lateral direction, see arrow D in Figure, of the intermediate component 40 with respect to the base component 20.

The second intermediate component 50 may be slidingly mounted with respect to the first intermediate component 40 in the axial direction x-x, for example within a seat 40a delimited by the first intermediate component 40, so as to enable to lengthen or shorten the distance of the apical component 30 (of the free end of apical component) from the base component 20. In this regard, cam means can be provided interceptable by the first intermediate component 40 during its medial shift, so as to determine the displacement of the second intermediate component 50 in the axial direction.

To return the centre of rotation to 0, the apical component 30, according to such embodiment, can be slidingly mounted with respect to the second intermediate component 50 so as to assume the position of the centres of rotation provided for the different lengths of the joint heads. By 0 it is intended the initial position of the device. In this regard, the apical component 30 may have a stem 30a slidingly mounted in a seat 50a delimited by the second intermediate component 50.

This device, as is shown in Fig. 4, can therefore be arranged with a standard ST, or offset or so-called "high offset" HOT or lateralized trim, in addition, of course, to the possibility of variation of the length of the joint heads.

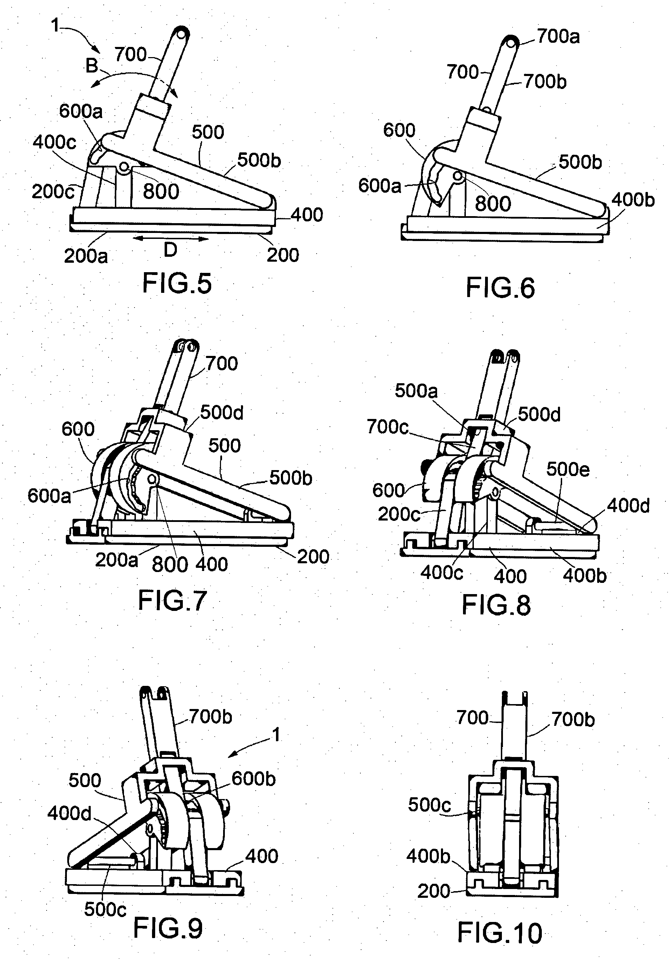

With reference now to Figures 5 to 10, another trial neck device 1 is illustrated according to the present invention comprising a base component 200 having, for example, a slide portion, while the first intermediate component 400 has, for example, a sliding block portion intended to slidingly engage in the slide portion of the base component 200, so as to allow sliding in the medial-lateral direction, see arrow D in Figure 5, of the first intermediate component 400 with respect to the base component 200.

A second intermediate component 500 is then provided, pivoted or pivotable with respect to the first intermediate component 400 about an axis, antero-posterior in use, so that the device according to Figures 5 to 10 can be arranged in different trims, with different neck-shaft angles (see arrow B).

More particularly, the first intermediate component 400 can have one or a pair of sliding block portions 400b, starting from one or each of which one or more respective stems or upright elements 400c extend, in use, either upwards or towards the cotyle. At the end of the upright elements 400c, distally from the respective sliding block 400b, one or more cam or desmodromic cam components 600 are pivoted. The cam component/s can delimits/delimit a suitably shaped seat 600a, for example extending substantially along a section or length of the circle with centre in the pivoting point of the cam component 600 relative to the respective upright element 400c, said seat 600a being however delimited by a pair of walls having portions or sections substantially inclined with respect to each other.

The second intermediate component 500 comprises instead one or more rod-like elements 500b pivoted, at one end, to the first intermediate component 400, for example to a respective sliding block 400b or to a lug element 400d fixed to it. The other end of the second intermediate component 500 is operatively connected to the cam component 600, for example starting from the other end of the second intermediate component 500 a small pin 500c extends, slidingly mounted within the or a respective seat 600a. Preferably, the second intermediate component 500 has two rod-like elements 500b connected by a bridge-type bracket 500d, optionally substantially U-shaped or with an intermediate U-shaped section, one end of the rod-like elements 500b being connected via a respective pin element 500e. The pin element 500e is pivoted to the first intermediate component 400, for example slidingly mounted within seats formed in the sliding blocks 400b or in lugs 400d extending from the same. Starting from the other end of the rod-like elements 500b, instead, the small pin 500c extends, slidingly mounted within a seat of one or more cam components 600. Naturally, another kind of kinematic connection could be envisaged, for example one or more rod-like elements could delimit a seat engageable by a respective pawl of the cam components.

The base component 200 may instead have a connecting rod or link element 200c, on one side, pivoted to a first lower portion, in use, 200a of the base component 200 and, at the other end, operatively connected to the cam component 600, for example having one or more pawls or cam follower rolls, each slidingly mounted in a respective seat 600a. Naturally, in this case, too, another type of kinematic connection could be provided.

In this device too, then, the apical component (not shown in the drawings) can be slidingly mounted with respect to the base component 200 so as to be movable apart and closer with respect to it. In this regard, the apical component will be fixed or connected to a third intermediate component 700, for example at its free end 700a, optionally fork-like. The third component 700 may have a top portion, in use, 700b, and a stem lower portion, in use, 700c, pivoted to the top portion 700b designed to slidingly engage in a seat 500a of an intermediate group, more particularly of the second intermediate component 500.

The lower stem portion 700c of the third intermediate component 700 is then pivoted to the cam components, for example a sleeve or shank 600b integral with the cam components 600, for example connecting the cam components 600.

In such neck device, by moving an element or component, a corresponding displacement is determined of other elements or components, taking into account that moving, for example, the cam components 600 a displacement is determined of the second and third intermediate component and, of course, of the apical component, while by moving, for example, the sliding block portions 400b of the second intermediate component 400 with respect to the base component 200, a displacement is determined of the small pins 500c and hence, of the cam components 600.

In this regard, the device may comprise a first engagement seat or recess for the insertion and operation of the adjustment means by means of a tool, such as a screwdriver or an Allen key or another means of transmission of motion. The first engagement seat is open in use towards the front and/or rear of the device, i.e. in the antero-posterior direction, so that it is engageable through a surgical tool starting from the front side of the patient, both for the case of a right hip and for the case of a left hip, and thus easily with a reduced joint, too, so as to avoid repeated joint dislocation, adjustment of the device and joint reduction manoeuvres. Two engagement seats can then be provided, one open towards the front and the other open towards the rear of the device.

In the event that the engagement seat is open towards the front and towards the rear, it is possible to adjust/use the device indifferently for the treatment of a right or a left hip.

According to the embodiment illustrated in Figures, a first engagement seat 800 can be formed substantially coaxial with the pivot pin of a cam component 600 to the first intermediate component 400.

A neck device according to the present invention is preferably configured substantially as the upper part of the femoral bone.

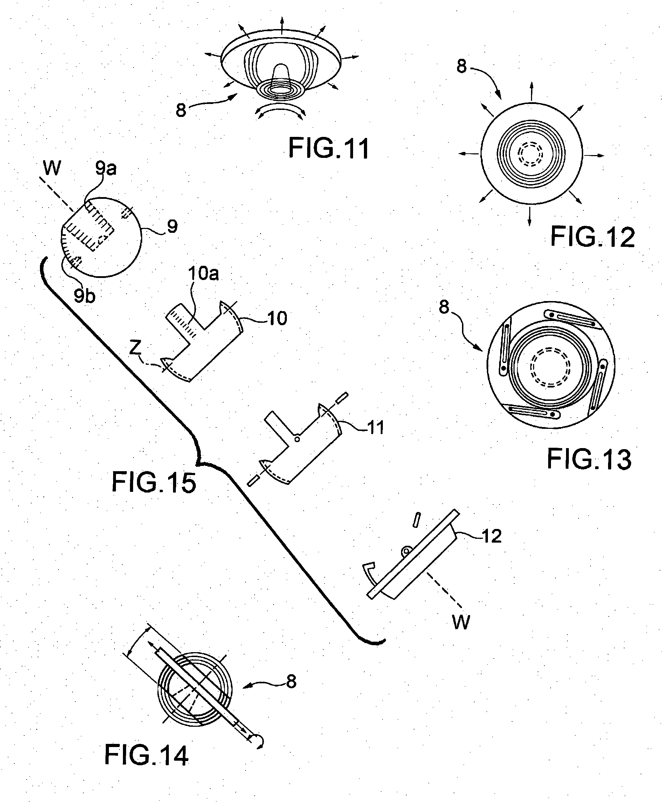

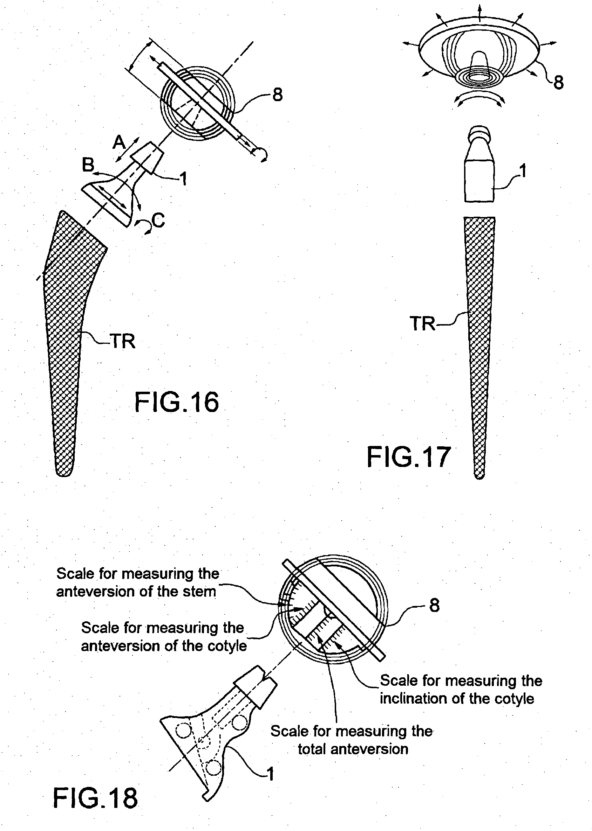

With reference now to Figures 11 to 22, a device 8 is illustrated for measuring the angle of femoral anteversion, the angle of anteversion of the acetabulum and or of the cotyle and/or of the acetabular and/or cotyloid inclination angle for the realization of a hip prosthesis according to the present invention, comprising at least a base or main body 9 mountable on an apical component of a trial neck device, for example on an apical component 3, 30 of a device 1 like the one described above, in this regard see Figures 16 to 18, in which a kit is illustrated according to the present invention comprising a device 1 and 8 during a respective phase of assembly.

The device 8 further comprises at least one auxiliary component engageable with the main component 9, as well as engagement means of the main component with the apical component 3, 30 along a longitudinal axis w-w, for example defining a housing seat 9a of the apical component 3, 30 extending about a longitudinal axis w-w.

The auxiliary component/s is/are displaceable with respect to the main component 9 about a transverse axis, for example orthogonal z-z to the longitudinal axis w-w and, preferably, means are then provided for measuring the displacement of the auxiliary component/s with respect to the base body.

The transverse axis z-z is designed to be located:
- on the front plane and in the inferior medial-lateral upper direction so as to measure the angle of anteversion of the femoral trial rasp or the implanted prosthetic femoral component or the acetabular and/or cotyle anteversion angle; and/or
- on the sagittal plane and in the antero-posterior direction, so as to measure the acetabular and/or cotyloid inclination angle.

More particularly, the base body may comprise a substantially curved outer surface, even more preferably, it can be configured as a sphere or part of a sphere, while the auxiliary component can be configured as a dome or shell mountable on and at least partially around the base body 9.

Each prosthetic cotyloid system of the hip provides different configurations of both the cotyle and the insert with the purpose of ensuring suitable angular ratios between the components such as to ensure the so-called "Safe Zone", the zone within which the centre of rotation of the femoral head of the patient must fall in order to ensure stability.

The device 8 is able to measure all the angles necessary to ensure compliance with the Safe Zone both working in conjunction with the device 1, and on a traditional trial neck or on any prosthetic femoral component, particularly where the latter present an apical component corresponding to a cone, for example the so-called "Morse cone 12-14", usually universally adopted, although, obviously, the device 8 can be adapted in the construction phase to Morse cones of different types.

With particular reference to the embodiment illustrated in Figures, the device 8 comprises a main component 9 configured as a sphere or part of a sphere and one or more, preferably three auxiliary portions 10, 11 and 12 configured as a shell or dome, for example spherical or in any case arranged or extending along a respective spherical surface, preferably with diameter increasing from the inner dome 10 to the outer dome 12 and intended to be mounted concentrically one over the other and so as to be able to mutually slide on one another concentrically.

The first dome or the inner dome 10 will be mounted on the main component 9 and will be pivoted or hinged to it, for example by a pin diametrically arranged along an axis z-z, preferably passing through the centre of the sphere itself and orthogonally to the longitudinal axis w-w. More particularly, such a pin is intended to be located, in use, on the front plane and in the inferior medial-lateral upper direction. The first dome 10 will thus be free to rotate only in the front and rear direction and can therefore measure the angle of anteversion of the femoral trial rasp or the implanted femoral prosthetic component. The measurement means may include graduated scales 9b imprinted on the main component 9 or on the first dome 10. Preferably, in its movement around the respective pin, the first dome 10 will drag with it the second 11 and third 12 dome too, since, preferably, the second dome 11 is integral with the first 10 and free to move only with respect to it, while the third dome 12 is, preferably, movable with the second and free to move only with respect thereto.

The second dome portion 11 will, preferably, be mounted on the first and hinged to this by the same pin between the first dome portion 10 and the main component 9, about the axis z-z, therefore also the second dome portion 11 can rotate only in the front and rear direction with respect to the first dome 10 and via the same it will therefore be possible to measure the angle of anteversion of the acetabulum and/or cotyle. The degrees referring to this angle can be detected on a suitable graduated scale 10a printed on the first 10 or the second 11 dome, while the sum of the two angles of anteversion will be detectable on a special graduated scale printed, for example, on the central sphere. The second dome during its movement about the respective hinge, will drag with it the third dome 12 too.

The third dome portion 12 will instead be mounted on the second 11 and hinged to this with one or more pins arranged diametrically and passing, in use, through the centre of rotation of the sphere and orthogonally to the longitudinal axis w-w on the sagittal plane and in the antero-posterior direction. The third dome will therefore preferably be free to rotate only in the lateral and the medial direction and orthogonally with respect to the first 10 and second 11 dome and can therefore measure the acetabular and/or cotyloid angle of inclination. The degrees of this angle can be detected on a suitable graduated scale printed indifferently on the second or the first dome or on the sphere.

The third dome 12 can then be provided with a first portion, for example, internal and a second portion, for example external that can be moved apart from/closer to the first portion, so as to increase or decrease the width of the auxiliary component to fit to cotyles of different sizes. To this regard, the third dome 12 may have a circular flange provided with a system of fins or flange portions which will expand as a result of actuation by suitable actuating means. These fins or flange portions by expanding will assume increasing diameters until coming into contact with the acetabular bone edge. This will be the ideal position of the prosthetic cotyloid component and will therefore be marked, for example with an electric scalpel, in order to be subsequently repeated.

The device 8 could also be provided or placed in electrical communication with infrared or telemetry means, data capture systems, robotised or computerised devices, etc.

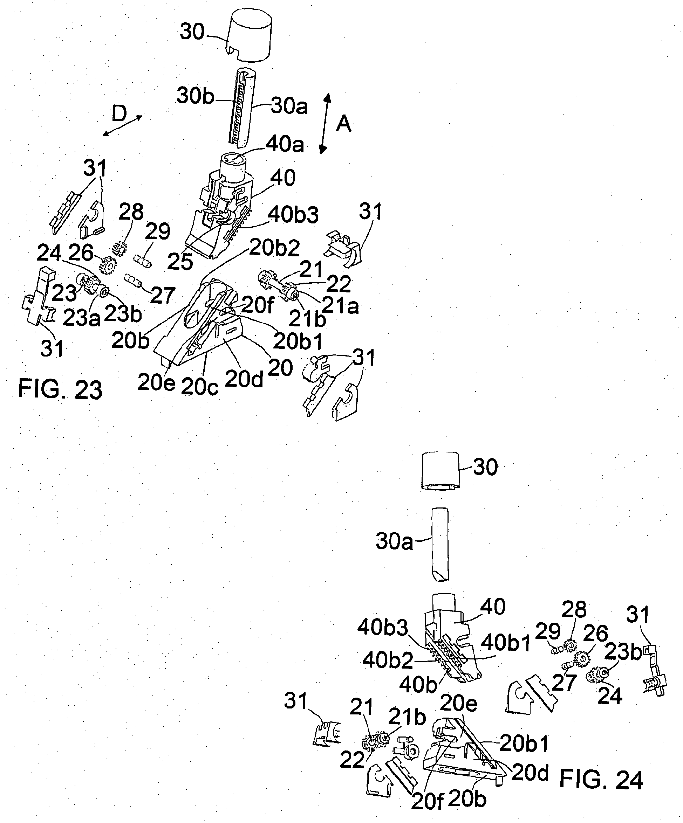

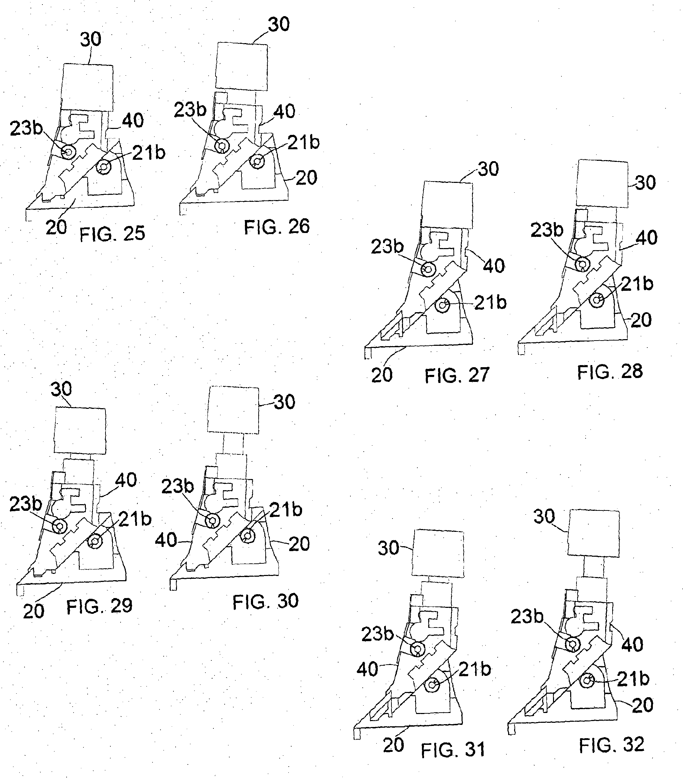

With reference now to Figures 23 to 32, they illustrate a trial neck device embodiment in accordance with the present invention and similar to the device of Figure 3.

A device according to this embodiment includes at least one pin rotatably mounted in a housing cradle delimited by one of the components or guiding component 20 or 30 or 40 as well as at least one gear wheel mounted or keyed on such pin, said pin ending at one of its ends in a head delimiting the engagement seat or recess. Another component or displaceable component 20 or 30 or 40 comprises instead a toothed section or portion for meshing engagement with a respective toothed wheel, so that controlling the rotation of the pin the engagement or sliding of the toothed section or portion, and therefore, the translation of the displaceable component with respect to the guiding component 20, 30, 40 is determined.

Such device 1 can comprise a base component 20 adaptable and fixable to a trial rasp TR or to a final prosthetic component, as well as an apical component 30 and a first intermediate component 40 fixed, on one end, to the base component 20 and, on the other, to the apical component 30.

More particularly, the base component 20 may have a slide portion 20b turned upwards, in use, while the first intermediate component 40 has a lower, in use, sliding block portion 40b, intended to slidingly engage with the slide portion 20b of the base component 20, so as to allow a shift in the medial-lateral direction, see arrow D in Figure, of the intermediate component 40 with respect to the base component 20. Furthermore, the sliding block portion 40b could also be such as to slidingly engage the slide portion 20b of the base component 20, so as to allow an adjustment of the distance (see arrow A in the Figure) of the apical component 3 with respect to the base component 2.

The adjustment of the mutual position in the medial-lateral direction between the base component 20 and intermediate component 40 can for example be obtained by means of an engagement of the rack and pinion type between the respective parts or sections of said components or integral with said components.

In this regard, the base component 20 may include a bottom wall 20c, if desired, substantially flat, a pair of main walls 20d, one at the front, in use, and the other at the rear, in use, extending from the bottom wall 20c and delimiting at the top the slide portion 20b. To this regard, the slide portion 20b may include two strip elements or sections 20b1, 20b2 spaced and, optionally, parallel, each corresponding to the top edge, in use, of a respective main wall 20d.

The strip sections 20b1, 20b2 may be, in use, horizontal or substantially inclined relative to the bottom face 20c of the base component 20 and to the vertical line, in use.

Furthermore, each main wall 20d may have an area or recessed portion 20e for slidingly housing, as will be discussed in more detail below, a respective toothed section of the first intermediate element 40. In this case, the strip sections 20b1, 20b2 protrude externally, or one in the front direction and the other in the rear direction with respect to the recessed area 20e.

A first pin 21 is then provided, rotatably mounted in a first housing cradle 20f delimited by the base component 20, for example extending transversely or orthogonally to the main walls 20d, for example, in the antero-posterior direction. The housing cradle 20f may be open to one side of the base component 20.

On the first pin 21 one or a pair of first wheels or toothed rings 22 is then mounted or keyed, intended to externally protrude, in use, from the area between the main walls 20d and more particularly within a recessed area or portion 20e of a respective main wall 20d. The first pin 21 terminates at one or both of its ends in a head 21a delimiting a second engagement seat or recess 21b for the insertion and the actuation of the adjustment means by means of a tool, such as a screwdriver or an Allen wrench or another means of transmission of motion. The second engagement seat 21b is open in use towards the front and/or rear of the device, i.e. in the antero-posterior direction, so that it is engageable through a surgical tool starting from the front side of the patient, both for the case of a right hip and for the case of a left hip, and thus easily also with a reduced joint, so as to avoid repeated joint dislocation, adjustment of the device and reduction joint manoeuvres.

The first intermediate component 40, instead, has a sliding block portion 40b as aforesaid, lower in use, which may comprise a pair of limb sections 40b1, 40b2, each designed to slidingly engage with a respective strip section 20b1, 20b2. The limb sections 40b1, 40b2 are projecting, in use, downwards, and are optionally parallel with one another and at a distance slightly greater than the two strip elements or sections 20b1, 20b2 of the base component 20. In this regard, the limb sections 40b1, 40b2 may have one L shaped end so as to slidingly wrap the strip sections 20b1, 20b2 of the slide portion 20b of the base component 20.

On the free face of the limb sections 40b1, 40b2, that is to say the face turned towards the base component 20, a toothed section 40b3 can be provided for meshing engagement with a respective toothed wheel 22.

Basically, controlling the rotation of the pin 21 by means of a tool inserted in the engagement seat 21b, it is possible to determine the engagement and the sliding of one or both toothed sections 40b3 of the first intermediate component and therefore the translation of the latter with respect to the base component 20 in the medial-lateral direction and, optionally, the adjustment of the distance (see arrow A in Figure) of the apical component 3 with respect to the base component 2.

The apical component 30 can instead be slidingly mounted with respect to the first intermediate component 40 so as to assume the position of the centres of rotation provided for the different lengths of the joint heads.

Furthermore, the apical component 30 may present a stem 30a slidingly mounted in a seat 40a delimited by the first intermediate component 40. In this regard, the stem 30a of the apical component 30 may be slidingly mounted with respect to the first intermediate component 40 in the axial direction x-x, for example within the seat 40a delimited by the first intermediate component 40, so as to enable to lengthen or shorten the distance of the apical component 30 (of the free end of the apical component) from the base component 20.

In this respect, the adjustment of the relative position in the axial direction between the apical component 30 and first intermediate component 40 and then between the apical component 30 and base component 20 can for example be obtained by means of an engagement of the rack and pinion type between the respective parts or sections of said components or integral with said components.

With reference to this aspect, in the first intermediate component 40 one or more second pins 23 can be mounted on which respective wheels or toothed sectors 24 are keyed, while the stem 30a of the apical component 30 has a toothed portion 30b intended to meshing engage one of said gear wheels 24, directly or by interposition (as will be described in greater detail below) of intermediate toothed elements. The second pin 23 could terminate at one or both of its ends in a head 23a with a third engagement seat or recess 23b for the engagement by means of a tool, such as a screwdriver or an Allen wrench or another means of transmission of motion. The third engagement seat 23b is, preferably, open in use towards the front and/or rear of the device, i.e. in the antero-posterior direction, so that it is engageable by a surgeon through a tool starting from the front side of the patient, both for the case of a right hip and for the case of a left hip, and thus easily also with a reduced joint, so as to avoid repeated joint dislocation, adjustment of the device and reduction joint manoeuvres.

According to the specific embodiment illustrated in the Figures, a second pin 23 is provided, rotatably mounted within a respective second housing cradle seat 25 delimited by the first intermediate component 40 and intended to engage by meshing a first pinion 26 mounted on a third pin 27 rotatably mounted in or with respect to the first intermediate component 40, which, in turn, is intended to engage by meshing a second pinion 28 mounted on a fourth pin 29, which is also rotatably mounted in the first intermediate component 40. The second pinion 28 is then intended to engage by meshing the second toothed section 30b of the stem 30a of the apical component 30.

The second pin 23, the third pin 27 and the fourth pin 29 are all substantially parallel and rotatably mounted about a respective axis parallel to an antero-posterior direction.

The device according to the embodiment of Figures 23 to 32 may then include masking elements 31 to conceal the respective components of the device.

The actuations and displacements described above, both with reference to the device 1 and to the device 8, may be obtained by any suitable adjustment means, such as a rack and pinion system, a worm screws and rack system, by means of simple or desmodromic cams so as to ensure, if necessary, the synchronisation of movements. Alternatively, the displacements could be controlled by means of a special key or brought to the outside of the surgical site with flexible cables or cardan shafts and actuated by means of sliders and or knobs on which the values of the different positions could also be read. Naturally, other means of adjustment could also be used, mechanical, hydraulic, electrical, magnetic, electronic or of another nature.

The inventions according to the present application are applicable to any prosthetic hip system, of any manufacturing firm and/or distribution commercial firm.

The inventions are furthermore applicable both for prosthetic systems that provide femoral components, monobloc, with modular joint heads, monolithic stems or femoral stems with modular necks.

The invention is applicable to any surgical technique, surgical procedure, way of access, traditional techniques, techniques involving the use of navigators or robotised techniques, separately or in conjunction.

The invention relates to devices 1 and 8, whether used separately or jointly.

The measurements of the angles in the devices according to the present invention can be performed with references, goniometers, or other suitable systems.

The devices according to the present invention can be used both to make measurements and addressing intraoperative and laboratory choices, and to make measurements and addressing operations during working of implant sites, as well as to carry out inspection and measurement of prosthetic implants.

The devices according to the present invention can be implemented in either a disposable, or a "reusable" version, in which case the two components will be sterilised after each use.

A device 1 according to the present invention enables to perform a single joint reduction manoeuvre, since the values of the neck-shaft angle, of the standard offset or high offset, of the length of the prosthetic neck and of the lengths of the joint heads, of the ante- or retroversion of said neck can be varied with a reduced joint, if desired, by actuating on the trial neck, with a suitable key, a system or group of levers designed to change the mutual position of the components of the devices and hence, all the parameters, thus replicating all the different options available of the femoral prosthetic system in use.

Basically, a method for the detection and/or measurement of the physical/structural characteristics of a bone joint of a patient for the implementation of a hip prosthesis according to the present invention includes the following steps:
- providing a device 1;
- dislocating a patient's joint;
- constraining the base component 2, 20, 200 to a trial rasp TR or a final prosthetic component;
- constraining the trial rasp TR or the final prosthetic component to the patient's femur;
- arranging the apical component 3, 30 proximally to the patient's cotyle;
- reducing the joint;
- actuating the adjustment means so as to adjust, with a reduced joint, the distance and/or the angular position of the apical component 3, 30 with respect to the base component 2, 20, 200; and
- dislocating the joint and removing the device.

The apical component 3, 30 could, prior to the implementation of the above described method, be inserted or grafted into an element with the outer surface at least partially spherical or curved, to be positioned within the cotyle of the patient, for example, within a device 8 or more particularly within a housing seat 9a of the latter.

Alternatively, the device can be structured in such a way that individual actuations are provided for each single configuration, with a second or other actuation intended to reproduce the different lengths of the joint head, so that, with extreme ease, accuracy and speed, the surgeon is able to explore all the possible solutions and choose the final configuration that best meets the required parameters.

In view of this, surgical intervention times are reduced as well as exposure of the part to be treated, which implies a reduction of the risk of surgical site infection and surgical joint damage, since several reduction and dislocation manoeuvres are avoided, resulting in greater minimal invasiveness, greater agility, safety and easier operation by the surgeon, in terms of choice of the final available configuration.

A device according to the present invention also allows the surgeon to operate using the so called "femur first" technique, which differs from standard techniques, in which first the cotyloid component is positioned, which is usually more susceptible to positioning errors, due to wear and possible arthrosis, and then, in a second phase, the femoral component, to then search for angular orientations of the cotyloid component, with a not ideal final result since a number of compromises must be accepted.

By contrast, according to the femur first technique, the femoral component is positioned first, and then, in a second phase, operation is carried out on the acetabulum, so as to position the cotyloid component with a suitable angular orientation, to position the device within the so-called "safe zone"; this orientation being obtained through the combination of anteversion of the femoral component and anteversion and inclination of the acetabular component, the joint is led to have an optimum range of motion as well as the best possible stability in a reduced condition, if not the complete elimination of the risk of post-surgical dislocations.

The difficulty in adopting the "femur first" technique faced with conventional devices is due to the practical impossibility to correctly and suitably measure, in the surgical field, the angular ratios between the femoral component and the cotyloid component before having carried out the definitive work, if not through the adoption and use of navigation techniques and or through robotised surgery techniques; such techniques, however, imply very high costs, longer surgical times, as well as the intervention of highly trained surgeons.

The invention solves these problems by ensuring an extremely easy, accurate and effective system, since it is already applicable to the initiating femoral rasp or the first rasp (since the operation is carried out by using several rasps of increasing size, and the orientations can be modified between the first and the last final rasp), thus enabling, even before the final preparation of the still adjustable femoral component, to know and accurately measure both the final configuration of the final femoral component and the angular ratios suitable and functional for the "safe zone" of the cotyloid component, enabling to choose the most suitable configuration of the prosthetic cotyloid component and or its insert, of the shoulder type or not, any protrusion, inclination, eccentric, etc., allowing the marking of suitable landmarks on the acetabular bone for the subsequent operation and implanting of the acetabular or cotyloid component.

This is achieved, as aforesaid, by mounting a device 8 on the adjustable trial neck, which device does not alter the final adoptable configuration, since it is constantly centred with respect to the centre of rotation of the joint according to the configuration in question.

This device enables the advancement and the completion of the operations with certainty of the final obtained result, allowing, however, for intermediate and final inspection both with the trial components, such as femoral stem and cotyle, and with the final implant components.

In short, the invention consists of two devices, one is related to the intraoperative choice of the various available configurations of the femoral component and enables to treat the patient with a single reduction and dislocation manoeuvre and can be used in all surgical techniques, while the second is intended to measure the angular ratios between the femoral prosthetic component and the cotyloid prosthetic component and can be used as an aid and guide in the intraoperative choice of the versions of the femoral prosthetic component in relation to the attainment of the "safe zone" and or as a measuring, operation and placement guide of the cotyloid prosthetic component in the "femur first" technique.

The invention can be built to adapt to all the existing configurations of all the existing prosthetic femoral systems as it is composed of elements that are articulated and move one with respect to the other, so as to reproduce the various positions of the joint centre of rotation provided by each system.

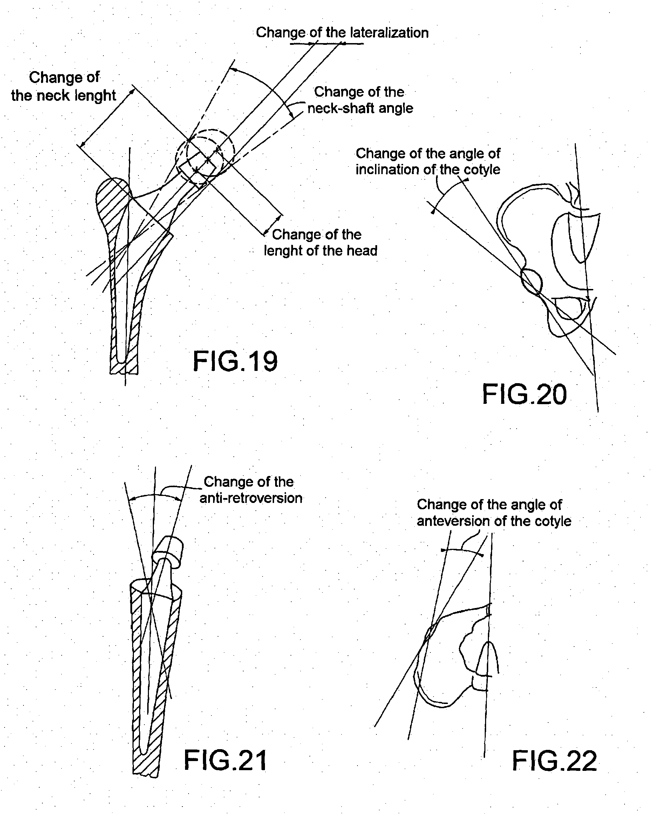

In the Figures 19 to 22 the measurable angles have been schematically shown with the devices according to the present invention.

The devices according to the present invention can be made disposable or reusable and can have control means, measurement means and data detection means of any type.

As it will be possible to ascertain, none of the devices hitherto proposed is structured as a trial neck device according hereto.

With reference for example to the devices taught by US7425214B1, US5645607A, US2004267372A1 and WO03094803A1, they do not allow adjustments with a reduced joint, since they have seats for adjustment tools in positions clearly covered or otherwise not accessible once the respective device has been implanted and the joint has been reduced or in any case, they have a structure not adjustable in a reduced joint condition.

Therefore, with such devices according to the prior art, the surgeon must adjust the components of the device, implant the respective device, reduce the joint and in the event that the adjustment carried out is not correct, the surgeon should dislocate the joint and re-adjust the device.

This, as was stated and described here above, is not necessary with a device according to the present invention.

Moreover, a device in accordance with the present invention comprises adjustment means to perform at least two of the following adjustments between apical component and base component: adjusting the relative distance (see Figures 1, 2 and 3), adjusting the angular position (see Figures 1 and 2) and adjusting the relative lateral-medial position (see Figure 3). This will clearly guarantee greater accuracy and rapidity in the adjustment phases.

Modifications and variations of the invention are possible within the scope of the appended claims.

## Claims

1. Trial neck device for detecting and/or measuring the physical/structural characteristics of a bone joint of a patient for making a hip prosthesis, said trial neck device comprising at least one base component (2, 20, 200) and at least one apical or terminal component (3, 30) constrained to said at least one base component (2, 20, 200) and moveable with respect to said at least one base component (2, 20, 200) so as to allow said detecting or said measuring, said at least one base component (2, 20, 200) being designed to be connected to a trial rasp (TR) or to a final prosthetic component intended to be constrained to the femur of a patient, whereas said at least one apical component (3, 30) being designed to be arranged proximal to the cotyle of the patient, wherein the device comprises adjustment means for adjusting the distance and/or the angular position of said at least one apical component (3, 30) with respect to said at least one base component (2, 20, 200), said adjustment means being operable with joint reduced, so that said device is suitable for detecting and/or measuring said physical/structural characteristics with joint reduced, **characterised in that** it comprises at least two of the following adjustment means: adjustment means for adjusting the distance of said apical component (3, 30) with respect to said at least one base component (2, 20, 200), adjustment means for adjusting the angular position of said at least one apical component (3, 30) with respect to said at least one base component (2, 20, 200) and adjustment means for adjusting the lateral-medial position of said at least one apical component (3, 30) with respect to said at least one base component (2, 20, 200).

2. Device according to claim 1, **characterised in that** said at least one apical component (3, 30) is slidably mounted with respect to said at least one base component (2, 20, 200) thereby being moveable closer and apart from it and, in use, towards and away from the cotyle.

3. Device according to any previous claim, **characterised in that** it comprises at least one intermediate group (4, 40, 50, 400, 500, 600, 700), constrained, on one side, to said base component (2, 20, 200) and, on the other side, to said apical component (3) thereby connecting said base component (2, 20, 200) to said apical component (3, 30).

4. Device according to claim 3, **characterised in that** said intermediate group (4, 40, 50, 400, 500, 600, 700) has at least one portion (4, 40, 50, 400, 700) designed to engage by sliding with a respective portion of said base component (2, 20, 200) and/or of said apical component (3, 30).

5. Device according to claim 4, **characterised in that** at least one between said at least one base component (2, 20, 200) and said intermediate group (4, 40, 400) has a slide portion (200a), whereas the other between said intermediate group (4, 40, 400) and said at least one base component (2, 20, 200) has a sliding block portion (400b) intended to slidably engage said slide portion (200a), thereby allowing a sliding in a medial-lateral direction of said intermediate group (4, 40, 400) with respect to said at least one base component (2, 20, 200).

6. Device according to claim 3, 4 or 5, **characterised in that** said intermediate group has at least one portion (4, 700) hinged to said base component (2, 20) and/or to said apical component (3, 30).

7. Device according to claim 6, **characterised in that** said intermediate group (4, 40, 400, 500, 600, 700) is hinged to said base component (2, 20, 200) and/or to said apical component (3, 30) about an axis substantially parallel to an antero-posterior direction, so as to assume different neck-shaft angles.

8. Device according to claim 6 or 7, **characterised in that** said intermediate group (4, 40, 400) is hinged to said base component (2, 20) and/or to said apical component (3, 30) about an axis substantially parallel to the lateral-medial direction, so as to assume different inclinations in the antero-posterior direction.

9. Device according to any one of the previous claims 3 to 8 when depending upon claim 3, **characterised in that** said intermediate group comprises at least one first intermediate component (400) and at least one second intermediate component (500), and **in that** it comprises at least one cam or desmodromic cam component (600) hinged to said first intermediate component (400), said second intermediate component (500) comprising at least one rod-like element (500b) hinged, at one end, to said first intermediate component (400), whereas its other end is operatively connected to said cam component (600).

10. Device according to claim 9, **characterised in that** said base component (200) has a connecting rod or link element (200c), hinged, on one side to said base component (200) and, on the other side, operatively connected to said cam component (600).

11. Device according to claim 9 or 10, **characterised in that** said intermediate group comprises a third intermediate component (700) having a top portion (700b) and a stem lower portion (700c) hinged to the top portion (700b) and designed to slidably engage in a seat (500a) of said second intermediate component (500), said stem lower portion (700c) being hinged to said at least one cam component (600).

12. Device according to any previous claim, wherein said device comprises at least one engagement seat or recess (800, 21b, 23b) for the insertion and the operation of said adjustment means through a tool, said engagement seat (800, 21b, 23b) being open, in use, towards the front and/or rear of said device, i.e. said engagement seat (800, 21b, 23b) extends about an axis in the antero-posterior direction.

13. Device according to any previous claim, wherein said adjustment means are selected from the group including a rack and pinion system, a worm screws and rack system, mechanical, hydraulic, electrical, magnetic or electronic adjustment means.

14. A device as claimed in claim 12 or 13, **characterized in that** it comprises at least one first (21) or a second (23) pin rotatably mounted in a housing cradle (20f, 25) delimited by one of the components or guiding component (20, 30, 40) of said device as well as a toothed wheel (22, 24) mounted or keyed on said at least one first (21) or second (23) pin, said at least one first (21) or second (23) pin terminating at at least one of its end in a head (21a, 23a) delimiting said engagement seat or recess (21b, 23b), and **in that** another of the components or displaceable component (20, 30, 40) of said device comprises at least one toothed section (40b3) or portion (30b) for meshing engaging a respective toothed wheel (22, 24), so that controlling the rotation of said least one first (21) or second (23) pin the engagement or sliding of at least one toothed section (40b3) or portion (30b), and therefore, the translation of said displaceable component (20, 30, 40) with respect to said guiding component (20, 30, 40) is determined.

15. Kit for making a hip prosthesis, comprising a trial neck device (1) according to any claim 1 to 14 as well as a measuring device (8) for measuring the angle of anteversion of the femoral component, the angle of anteversion of the acetabulum and/or of the cotyle and/or the angle of the acetabular and/or the cotyloid inclination for making a hip prosthesis, comprising:
- at least one main component (9) mountable on said apical component (3, 30) of said trial neck device;
- at least one auxiliary component (10, 11, 12) engageable with said main component (9); and
- engagement means of said main component (9) with said apical component (3, 30) along a longitudinal axis (w-w), said at least one auxiliary component (10, 11, 12) being movable with respect to said main component (9) about an axis transversal to said longitudinal axis (w-w).

## Patentansprüche

1. Probehalsvorrichtung zum Erfassen und/oder Messen der physikalisch/strukturellen Eigenschaften eines Knochengelenks eines Patienten zur Herstellung einer Hüftprothese, wobei die besagte Probehalsvorrichtung mindestens eine Basiskomponente (2, 20, 200) und mindestens eine apikale oder terminale Komponente (3, 30) umfasst, die an der besagten mindestens eine Basiskomponente (2, 20, 200) festgehalten wird und in Bezug auf die besagte mindestens eine Basiskomponente (2, 20, 200) beweglich ist, um das besagte Erfassen oder das besagte Messen zu ermöglichen, wobei die besagte mindestens eine Basiskomponente (2, 20, 200) dazu ausgelegt ist, mit einer Proberaspel (TR) oder mit einer endgültigen prothetischen Komponente verbunden zu werden, die dazu bestimmt ist, an dem Oberschenkelknochen eines Patienten festgehalten zu werden, wohingegen die besagte mindestens eine apikale Komponente (3, 30) dazu ausgelegt ist, proximal zur Gelenkpfanne des Patienten angeordnet zu werden, worin die Vorrichtung Einstellmittel zum Einstellen des Abstandes und/oder der Winkelposition der besagten mindestens einen apikalen Komponente (3, 30) in Bezug auf die besagte mindestens eine Basiskomponente (2, 20, 200) umfasst, wobei die besagten Einstellmittel mit reduziertem Gelenk betreibbar sind, sodass die besagte Vorrichtung zum Erfassen und/oder Messen der besagten physikalischen/strukturellen Eigenschaften mit reduziertem Gelenk geeignet ist, **dadurch gekennzeichnet, dass** sie mindestens zwei der folgenden Einstellmittel umfasst: Einstellmittel zum Einstellen des Abstandes der besagten apikalen Komponente (3, 30) in Bezug auf die besagte mindestens eine Basiskomponente (2, 20, 200), Einstellmittel zum Einstellen der Winkelposition der besagten mindestens einen apikalen Komponente (3, 30) in Bezug auf die besagte mindestens eine Basiskomponente (2, 20, 200) und Einstellmittel zum Einstellen der lateral-medialen Position der besagten mindestens einen apikalen Komponente (3, 30) in Bezug auf die besagte mindestens eine Basiskomponente (2, 20, 200).

2. Vorrichtung nach Anspruch 1, **dadurch gekennzeichnet, dass** die besagte mindestens eine apikale Komponente (3, 30) in Bezug auf die besagte mindestens eine Basiskomponente (2, 20, 200) verschiebbar montiert ist, wodurch sie daran angenähert und davon entfernt und, bei Verwendung, zur Gelenkpfanne hin und davon weg bewegbar ist.

3. Vorrichtung nach irgendeinem vorangegangenen Anspruch, **dadurch gekennzeichnet, dass** sie mindestens eine Zwischengruppe (4, 40, 50, 400, 500, 600, 700) umfasst, die auf der einen Seite an der besagten Basiskomponente (2, 20, 200) und auf der anderen Seite an der besagten apikalen Komponente (3) festgehalten wird, wodurch die besagte Basiskomponente (2, 20, 200) mit der besagten apikalen Komponente (3, 30) verbunden wird.

4. Vorrichtung nach Anspruch 3, **dadurch gekennzeichnet, dass** die besagte Zwischengruppe (4,40, 50, 400, 500, 600, 700) mindestens einen Abschnitt (4, 40, 50, 400, 700) aufweist, der dazu ausgelegt ist, durch Gleiten in einen entsprechenden Abschnitt der besagten Basiskomponente (2, 20, 200) und/oder der besagten apikalen Komponente (3, 30) einzugreifen.

5. Vorrichtung nach Anspruch 4, **dadurch gekennzeichnet, dass** mindestens eine zwischen der besagten mindestens einen Basiskomponente (2, 20, 200) und der besagten Zwischengruppe (4, 40, 400) einen Gleitabschnitt (200a) aufweist, wohingegen die andere zwischen der besagten Zwischengruppe (4, 40, 400) und der besagten mindestens einen Basiskomponente (2), 20, 200) einen Gleitblockabschnitt (400b) aufweist, der für den gleitenden Eingriff mit dem besagten Gleitabschnitt (200a) bestimmt ist, wodurch ein Gleiten in eine medial-laterale Richtung der besagten Zwischengruppe (4, 40, 400) in Bezug auf die besagte mindestens eine Basiskomponente (2, 20, 200) ermöglicht wird.

6. Vorrichtung nach Anspruch 3, 4 oder 5, **dadurch gekennzeichnet, dass** die besagte Zwischengruppe mindestens einen Abschnitt (4, 700) aufweist, der an der besagten Basiskomponente (2, 20) und/oder an der besagten apikalen Komponente (3, 30) gelenkig angeordnet ist.

7. Vorrichtung nach Anspruch 6, **dadurch gekennzeichnet, dass** die besagte Zwischengruppe (4, 40, 400, 500, 500, 600, 700) an der besagten Basiskomponente (2, 20, 200) und/oder an der besagten apikalen Komponente (3, 30) um eine Achse im Wesentlichen parallel zu einer antero-posterioren Richtung gelenkig angeordnet ist, um unterschiedliche Hals-Schaft-Winkel einzunehmen.

8. Vorrichtung nach Anspruch 6 oder 7, **dadurch gekennzeichnet, dass** die besagte Zwischengruppe (4, 40, 400) an der besagten Basiskomponente (2, 20) und/oder an der besagten apikalen Komponente (3, 30) um eine Achse im Wesentlichen parallel zur lateral-medialen Richtung gelenkig angeordnet ist, um unterschiedliche Neigungen in der antero-posterioren Richtung einzunehmen.

9. Vorrichtung nach irgendeinem der vorangegangenen Ansprüche 3 bis 8, wenn abhängig von Anspruch 3, **dadurch gekennzeichnet, dass** die besagte Zwischengruppe mindestens eine erste Zwischenkomponente (400) und mindestens eine zweite Zwischenkomponente (500) umfasst, und dadurch, dass sie mindestens eine Nocken- oder desmodromische Nockenkomponente (600) umfasst, die an der besagten ersten Zwischenkomponente (400) gelenkig angeordnet ist, wobei die besagte zweite Zwischenkomponente (500) mindestens ein stabförmiges Element (500b) umfasst, das an einem Ende an der besagten ersten Zwischenkomponente (400) gelenkig angeordnet ist, wohingegen ihr anderes Ende mit der besagten Nockenkomponente (600) wirkverbunden ist.

10. Vorrichtung nach Anspruch 9, **dadurch gekennzeichnet, dass** die besagte Basiskomponente (200) ein Stab- oder Verbindungselement (200c) aufweist, das auf der einen Seite an der besagten Basiskomponente (200) gelenkig angeordnet und auf der anderen Seite mit der besagten Nockenkomponente (600) wirkverbunden ist.

11. Vorrichtung nach Anspruch 9 oder 10, **dadurch gekennzeichnet, dass** die besagte Zwischengruppe eine dritte Zwischenkomponente (700) mit einem oberen Abschnitt (700b) und einem unteren Schaftabschnitt (700c) umfasst, der an dem besagten oberen Abschnitt (700b) gelenkig angeordnet ist und für den gleitenden Eingriff in einen Sitz (500a) der besagten zweiten Zwischenkomponente (500) ausgelegt ist, wobei der besagte untere Schaftabschnitt (700c) an der mindestens einen Nockenkomponente (600) gelenkig angeordnet ist.

12. Vorrichtung nach irgendeinem vorangegangenen Anspruch, worin die besagte Vorrichtung mindestens einen Eingriffssitz oder eine Aussparung (800, 21b, 23b) zum Einsetzen und Betätigen der besagten Einstellmittel durch ein Werkzeug umfasst, wobei der besagte Eingriffssitz (800, 21b, 23b) bei Verwendung zur Vorderseite und/oder Rückseite der besagten Vorrichtung hin offen ist, d.h. dass sich der besagte Eingriffssitz (800, 21b, 23b) um eine Achse in die antero-posteriore Richtung erstreckt.

13. Vorrichtung nach irgendeinem vorangegangenen Anspruch, worin die besagten Einstellmittel ausgewählt sind aus der Gruppe bestehend aus einem Zahnstangen- und Ritzelsystem, einem Schneckenschrauben- und Zahnstangensystem, mechanischen, hydraulischen, elektrischen, magnetischen oder elektronischen Einstellmitteln.

14. Vorrichtung nach Anspruch 12 oder 13, **dadurch gekennzeichnet, dass** sie mindestens einen ersten (21) oder einen zweiten (23) Stift umfasst, der drehbar in einem Gehäusegestell (20f, 25) montiert ist, das durch eine der Komponenten oder eine Führungskomponente (20, 30, 40) der besagten Vorrichtung begrenzt ist, sowie ein Zahnrad (22, 24), das auf dem besagten mindestens einen ersten (21) oder zweiten (23) Stift montiert oder verkeilt ist, wobei der besagte mindestens eine erste (21) oder zweite (23) Stift mindestens eines seiner Enden in einem Kopf (21a, 23a) beendet, der den besagten Eingriffssitz oder die besagte Aussparung (21b, 23b) begrenzt, und dadurch, dass eine andere der Komponenten oder eine verschiebbare Komponente (20, 30, 40) der besagten Vorrichtung mindestens einen Zahnabschnitt (40b3) oder einen Abschnitt (30b) für den Zahneingriff in ein jeweiliges Zahnrad (22, 24) umfasst, sodass durch Steuern der Drehung des besagten mindestens einen ersten (21) oder zweiten (23) Stiftes das Eingreifen oder Gleiten mindestens eines Zahnabschnitts (40b3) oder Abschnitts (30b) und folglich die Verschiebung der besagten verschiebbaren Komponente (20, 30, 40) in Bezug auf die besagte Führungskomponente (20, 30, 40) bestimmt wird.

15. Kit zur Herstellung einer Hüftprothese, umfassend eine Probehalsvorrichtung (1) nach irgendeinem Anspruch 1 bis 14 sowie eine Messvorrichtung (8) zum Messen des Anteversionswinkels der femoralen Komponente, des Anteversionswinkels der Hüftgelenkpfanne und/oder der Gelenkpfanne und/oder des Winkels der Hüftgelenkpfannen- und/oder der Gelenkpfannenneigung zur Herstellung einer Hüftprothese, umfassend:
- mindestens eine Hauptkomponente (9), die an der besagten apikalen Komponente (3, 30) der besagten Probehalsvorrichtung oder montierbar ist;
- mindestens eine Hilfskomponente (10, 11, 12), die mit der besagten Hauptkomponente (9) in Eingriff bringbar ist; und
- Eingriffsmittel der besagten Hauptkomponente (9) mit der besagten apikalen Komponente (3, 30) entlang einer Längsachse (w-w), wobei die besagte mindestens eine Hilfskomponente (10, 11, 12) in Bezug auf die besagte Hauptkomponente (9) um eine Achse quer zu der besagten Längsachse (w-w) bewegbar ist.

## Revendications

1. Dispositif de cou d'essai pour détecter et/ou mesurer les caractéristiques physiques/structurelles d'une articulation osseuse d'un patient pour réaliser une prothèse de hanche, ledit dispositif de cou d'essai comprenant au moins un composant de base (2, 20, 200) et au moins un composant apical ou terminal (3, 30) maintenu sur ledit au moins un composant de base (2, 20, 200) et mobile par rapport audit au moins un composant de base (2, 20, 200) de façon à permettre ladite détection ou ladite mesure, ledit au moins un composant de base (2, 20, 200) étant conçu pour être raccordé à une râpe d'essai (TR) ou à un composant prothétique final destiné à être maintenu sur le fémur d'un patient, alors que ledit au moins un composant apical (3, 30) est conçu pour être disposé à proximité du cotyle du patient, dans lequel le dispositif comprend des moyens d'ajustement pour ajuster la distance et/ou la position angulaire dudit au moins un composant apical (3, 30) par rapport audit au moins un composant de base (2, 20, 200), lesdits moyens d'ajustement étant utilisables avec une articulation réduite, de telle sorte que ledit dispositif soit adapté pour détecter et/ou mesurer lesdites caractéristiques physiques/structurelles avec l'articulation réduite, **caractérisé en ce qu'**il comprend au moins deux des moyens d'ajustement suivants : des moyens d'ajustement pour ajuster la distance dudit composant apical (3, 30) par rapport audit au moins un composant de base (2, 20, 200), des moyens d'ajustement pour ajuster la position angulaire dudit au moins un composant apical (3, 30) par rapport audit au moins un composant de base (2, 20, 200) et des moyens d'ajustement pour ajuster la position latérale-médiane dudit au moins un composant apical (3, 30) par rapport audit au moins un composant de base (2, 20, 200) .

2. Dispositif selon la revendication 1, **caractérisé en ce que** ledit au moins un composant apical (3, 30) est monté de manière coulissante par rapport audit au moins un composant de base (2, 20, 200), de façon à être mobile plus près et séparément de celui-ci et, pendant l'utilisation, en direction et en éloignement du cotyle.

3. Dispositif selon l'une des revendications précédentes, **caractérisé en ce qu'**il comprend au moins un groupe intermédiaire (4, 40, 50, 400, 500, 600, 700), maintenu, sur un côté, avec ledit composant de base (2, 20, 200) et, sur l'autre côté, avec ledit composant apical (3), raccordant ainsi ledit composant de base (2, 20, 200) audit composant apical (3, 30).

4. Dispositif selon la revendication 3, **caractérisé en ce que** ledit groupe intermédiaire (4,40, 50, 400, 500, 600, 700) comporte au moins une partie (4, 40, 50, 400, 700) conçue pour s'accoupler en coulissant avec une partie respective dudit composant de base (2, 20, 200) et/ou dudit composant apical (3, 30).

5. Dispositif selon la revendication 4, **caractérisé en ce qu'**au moins un parmi ledit au moins un composant de base (2, 20, 200) et ledit groupe intermédiaire (4, 40, 400) comporte une partie de coulisseau (200a), alors que l'autre parmi ledit groupe intermédiaire (4, 40, 400) et ledit au moins un composant de base (2, 20, 200) comporte une partie de bloc coulissant (400b) conçue pour s'accoupler de manière coulissante avec ladite partie de coulisseau (200a), permettant ainsi un coulissement dans une direction médiane-latérale dudit groupe intermédiaire (4, 40, 400) par rapport audit au moins un composant de base (2, 20, 200).

6. Dispositif selon la revendication 3, 4 ou 5, **caractérisé en ce que** ledit groupe intermédiaire comporte au moins une partie (4, 700) articulée sur ledit composant de base (2, 20) et/ou ledit composant apical (3, 30).

7. Dispositif selon la revendication 6, **caractérisé en ce que** ledit groupe intermédiaire (4, 40, 400, 500, 600, 700) est articulé sur ledit composant de base (2, 20, 200) et/ou ledit composant apical (3, 30) autour d'un axe sensiblement parallèle à une direction antéropostérieure, de façon à prendre différents angles cou-axe.

8. Dispositif selon la revendication 6 ou 7, **caractérisé en ce que** ledit groupe intermédiaire (4, 40, 400) est articulé sur ledit composant de base (2, 20) et/ou ledit composant apical (3, 30) autour d'un axe sensiblement parallèle à la direction latérale-médiane, de façon à prendre différentes inclinaisons dans la direction antéropostérieure.

9. Dispositif selon l'une quelconque des revendications précédentes 3 à 8 quand elle dépend de la revendication 3, **caractérisé en ce que** ledit groupe intermédiaire comprend au moins un premier composant intermédiaire (400) et au moins un deuxième composant intermédiaire (500), et **en ce qu'**il comprend au moins un composant de came ou de came desmodromique (600) articulé sur ledit premier composant intermédiaire (400), ledit deuxième composant intermédiaire (500) comprenant au moins un élément semblable à une tige (500b) articulé, à une extrémité, sur ledit premier composant intermédiaire (400), alors que son autre extrémité est raccordée opérationnellement audit composant de came (600).

10. Dispositif selon la revendication 9, **caractérisé en ce que** ledit composant de base (200) comporte une tige de raccordement ou un élément de liaison (200c), articulé, sur un côté audit composant de base (200) et, sur l'autre côté, raccordé opérationnellement audit composant de came (600).

11. Dispositif selon la revendication 9 ou 10, **caractérisé en ce que** ledit groupe intermédiaire comprend un troisième composant intermédiaire (700) comportant une partie supérieure (700b) et une partie inférieure de tige (700c) articulée sur la partie supérieure (700b) et conçue pour s'accoupler de manière coulissante dans un siège (500a) dudit deuxième composant intermédiaire (500), ladite partie inférieure de tige (700c) étant articulée sur ledit au moins un composant de came (600).

12. Dispositif selon l'une des revendications précédentes, dans lequel ledit dispositif comprend au moins un siège d'accouplement ou renfoncement (800, 21b, 23b) pour l'insertion et le fonctionnement desdits moyens d'ajustement par l'intermédiaire d'un outil, ledit siège d'accouplement (800, 21b, 23b) étant ouvert, pendant l'utilisation, vers l'avant et/ou l'arrière dudit dispositif, c'est-à-dire que ledit siège d'accouplement (800, 21b, 23b) s'étend autour d'un axe dans la direction antéropostérieure.

13. Dispositif selon l'une des revendications précédentes, dans lequel lesdits moyens d'ajustement sont sélectionnés parmi le groupe comprenant un système de crémaillère, un système de vis sans fin et de crémaillère, des moyens d'ajustement mécaniques, hydrauliques, électriques, magnétiques ou électroniques.

14. Dispositif selon la revendication 12 ou 13, **caractérisé en ce qu'**il comprend au moins un premier (21) ou un deuxième (23) axe monté de manière rotative dans un berceau de logement (20f, 25) délimité par l'un des composants ou composant de guidage (20, 30, 40) dudit dispositif ainsi qu'une roue dentée (22, 24) montée ou clavetée sur ledit au moins un premier (21) ou deuxième (23) axe, ledit au moins un premier (21) ou deuxième (23) axe se terminant à au moins l'une de ses extrémités dans une tête (21a, 23a) délimitant ledit siège d'accouplement ou renfoncement (21b, 23b), et **en ce qu'**un autre des composants ou composant déplaçable (20, 30, 40) dudit dispositif comprend au moins une section dentée (40b3) ou partie dentée (30b) pour un engrènement avec une roue dentée (22, 24) respective, de telle sorte qu'en contrôlant la rotation dudit au moins premier (21) ou deuxième (23) axe, l'accouplement ou le coulissement d'au moins une section dentée (40b3) ou partie dentée (30b), et par conséquent, la translation dudit composant déplaçable (20, 30, 40) par rapport audit composant de guidage (20, 30, 40) est déterminée.

15. Kit pour réaliser une prothèse de hanche, comprenant un dispositif de cou d'essai (1) selon l'une quelconque des revendications 1 à 14 ainsi qu'un dispositif de mesure (8) pour mesurer l'angle d'antéversion du composant fémoral, l'angle d'antéversion de la cavité cotyloïdienne et/ou du cotyle et/ou l'angle d'inclinaison acétabulaire et/ou cotyloïde pour réaliser une prothèse de hanche, comprenant :
- au moins un composant principal (9) montable sur ledit composant apical (3, 30) dudit dispositif de cou d'essai ;
- au moins un composant auxiliaire (10, 11, 12) pouvant être accouplé avec ledit composant principal (9) ; et
- des moyens d'accouplement dudit composant principal (9) avec ledit composant apical (3, 30) le long d'un axe longitudinal (w-w), ledit au moins un composant auxiliaire (10, 11, 12) étant mobile par rapport audit composant principal (9) autour d'un axe transversal audit axe longitudinal (w-w).
